# FASCICULE DE BREVET EUROPEEN

(11) **EP 3 005 891 B1**
(45) Date de publication et mention de la délivrance du brevet: **26.04.2017**
(21) Numéro de dépôt: 15188893.0
(22) Date de dépôt: 08.10.2015
(51) Int. Cl.: A61F 5/00, A41D 13/00, A45F 3/14

(54) **VÊTEMENT POUR TRAVAUX DE MANUTENTION**
KLEIDUNGSSTÜCK FÜR VERLADEARBEITEN
GARMENT FOR HANDLING WORK

(30) Priorité: 08.10.2014 FR 1459635
(43) Date de publication de la demande: 13.04.2016
(73) Titulaire: Sidect, 04110 Villemus (FR)
(72) Inventeur: LABBE-LAINE, Yves, 83560 VINON-SUR-VERDON (FR)
(74) Mandataire: Roman, Alexis

(56) Documents cités:
- DE-A1- 19 800 727
- FR-A1- 3 001 382
- US-A1- 2013 104 280

## Description

### Domaine technique de l'inventon.

L'invention a pour objet un vêtement pour travaux de manutention, et plus particulièrement un vêtement permettant d'aider l'utilisateur lors de manipulations répétitives de charges.

Elle concerne le domaine technique des équipements qui apportent une aide et un soulagement à leur utilisateur, notamment dans le cas de travaux de manutention de charges, quelles qu'elles soient, mais aussi dans le cas de travaux impliquant une position durablement inclinée du tronc et, en particulier, lors de travaux manuels répétitifs réalisés, par exemple, par des manutentionnaires.

### État de la technique.

Les traumatismes au niveau des bras et du dos sont toujours présents malgré l'allègement des produits à manipuler. Malgré tout l'investissement apporté au niveau de la formation des personnes concernées par ces travaux de manutention, notamment la formation concernant les bons gestes et les bonnes postures, ces traumatismes sont encore fortement handicapants pour les professionnels de la manutention. Dans le domaine du BTP, de l'agriculture, 80% des travailleurs, salariés ou non, sont concernés par les traumatismes musculosquelettiques (TMS) ; ils souffrent notamment du dos et des bras.

Des équipements supplétifs individuels sont connus de l'art antérieur et par exemple décrits dans les documents brevets WO 2010/058046 (PALANCO), EP 1.264.583 (PRACHER), FR 2.864.891 (BROCHET), US 1.553.874 (NIVENS), US 6.129.691 (RUPPERT) ou encore US 6.191.342 (TAYLOR). Les équipements proposés peuvent apporter une aide et un soulagement à leur utilisateur. Ils sont constitués d'une structure faisant office de harnais de poitrail, lequel harnais comprend des éléments de maintien dorsaux et des éléments de maintien ventraux. Des sangles dorsales élastiques peuvent éventuellement s'étendre dans le dos de l'utilisateur, depuis le harnais de poitrail jusqu'aux jambes sur lesquelles elles sont destinées à être fixées.

Bien que de tels équipements donnent des forces complémentaires à l'organisme et, de ce fait, le soulagent durablement, ils n'apportent aucun soulagement au niveau des bras de l'utilisateur. Bien au contraire, les bras vont souffrir encore plus du fait des améliorations apportées au niveau du dos de l'utilisateur. En effet, l'amélioration des conditions de travail et du rendement, procurée par l'équipement supplétif, va entraîner un accroissement du travail et des efforts encaissés par les bras. L'amélioration gagnée au niveau du dos, entraîne une forte dégradation au niveau des bras.

Le document brevet FR 3.001.382 (SIDECT) propose un équipement supplétif permettant de pallier les inconvénients précités et présentant une structure qui fait office de harnais. Elle comprend des éléments de maintien dorsaux et ventraux qui permettent de soulager les bras de l'utilisateur lors de manutention de charges.

Bien que l'équipement proposé dans le document brevet SIDECT représente une amélioration des équipements proposés précédemment, ce système demeure peu confortable pour l'utilisateur. De plus, mis à part son manque d'esthétisme, la présence des différentes sangles dorsales et frontales comme éléments de maintien peut représenter un danger lors d'une utilisation en milieu complexe. Elles peuvent ainsi s'accrocher à des palettes, des poteaux, etc...représentant ainsi un réel danger pour son utilisateur.

Des vêtements supplétifs sont connus de l'état de la technique, et permettent de pallier à certains des inconvénients précités. Par exemple, e document brevet DE 19800727 (FAKLER) propose une veste comportant deux manches et un gilet. Le gilet se compose d'un pan dorsal et d'un pan ventral reliés aux manches par des emmanchures. La veste comprend un aménagement agencé sur chacune des manches de manière à former une ligne de tension entre le moyen d'accroche et le pan dorsal.

De la même manière, le document brevet US 2013/0104280 (BOYNTON) présente une veste similaire à celle décrite dans le document brevet DE 19800727 (FAKLER). Toutefois, les vêtements proposés comportent tous des systèmes de sangles complexes et difficiles à fabriquer et/ou installer sur les vestes. En effet, chacun des systèmes proposés présente des sangles venant se solidariser au niveau des poignets et étant raccordées de manière compliquée au niveau du pan dorsal.

L'invention vise à remédier à cet état des choses. En particulier, un objectif de l'invention est de proposer un vêtement pour travaux de manutention simplifié pouvant être installé de manière simple.

Un autre objectif de l'invention est de proposer un vêtement pour travaux de manutention dont la fabrication est simplifiée, réduisant ainsi les coûts de revient.

Encore un autre objectif de l'invention est de proposer un équipement pour travaux de manutention qui soit à la fois plus confortable à porter et plus esthétique.

### Divulgation de l'invention.

La solution proposée par l'invention est un vêtement pour travaux de manutention consistant en une veste comportant deux manches et un gilet, lequel gilet se compose d'un pan ventral et d'un pan dorsal reliés auxdites manches par des emmanchures, lesdites manches comportant chacune une extrémité pourvue d'un moyen d'accroche permettant leur solidarisation avec chacune des mains d'un utilisateur, ledit vêtement comprend au moins un aménagement agencé au niveau de chaque manche de manière à former une ligne de tension entre le moyen d'accroche et le pan dorsal, de sorte que ladite ligne de tension, en usage, oppose et/ou limite l'allongement du bras de l'utilisateur portant ledit vêtement.

Ce vêtement est remarquable en ce que et l'aménagement se compose d'au moins une sangle comportant une extrémité distale solidarisée à la manche, juste en dessous de l'emmanchure, et une extrémité proximale aménagée sur le pan dorsal, de sorte qu'une partie de la ligne de tension soit réalisée grâce à la mise en tension du tissu de la manche.

Le vêtement proposé par l'invention permet à l'utilisateur d'effectuer des travaux de manutention en sécurité et avec un doublement du bras lui permettant de soulever des charges importantes en évitant et/ou limitant les effets indésirables sur les bras et/ou les lombaires. De plus, la fabrication d'un tel vêtement est particulièrement simple et peut être adaptée à des vestes et/ou gilets préexistants.

D'autres caractéristiques avantageuses de l'invention sont listées ci-dessous. Chacune de ces caractéristiques peut être considérée seule ou en combinaison avec les caractéristiques remarquables définies ci-dessus, lesquelles caractéristiques remarquables ne sont pas indispensables en tant que telles à la résolution des problèmes techniques que les caractéristiques ci-dessous se proposent de résoudre :
- l'extrémité proximale de la sangle peut être rattachée au pan dorsal, au niveau des flancs de l'utilisateur, de sorte qu'une ligne de tension se créée lorsque l'utilisateur se courbe,
- la sangle peut être glissée dans un fourreau aménagé sur le pan dorsal, de manière à ce que ladite sangle passe sous les omoplates et au niveau des flancs de l'utilisateur,
- plusieurs fourreaux peuvent être aménagés sur le pan dorsal,
- la sangle peut être élastique,
- en usage, l'aménagement peut être configuré de sorte que la ligne de tension agit au niveau du pan dorsal de manière à obtenir une répartition dorsale de la charge autour et/ou à proximité de l'axe de gravité (Xg) de l'utilisateur,
- le moyen d'accroche peut présenter une boucle adaptée pour se coincer sous le pouce de l'utilisateur,
- la boucle peut se composer d'au moins une sangle élastique,
- la boucle peut être dimensionnée de manière à pouvoir passer autour de l'avant bras et/ou du bras de l'utilisateur,
- le matériau constituant la manche et/ou le pan dorsal au niveau de la ligne de tension peut avoir des propriétés élastiques différentes de celles du matériau constituant le reste du vêtement en dehors de ladite ligne de tension,
- le matériau constituant la manche et le pan dorsal au niveau de la ligne de tension peut être moins élastique que le matériau constituant le reste du vêtement en dehors de ladite ligne de tension,
- la sangle peut être directement intégrée dans la veste lors du tricotage dudit vêtement,
- la sangle, le long de la ligne de tension, peut posséder des propriétés élastiques différentes de celles du reste dudit vêtement,
- la sangle peut être formée de mailles et/ou de fils différents de ceux utilisés pour le reste dudit vêtement,
- le matériau constituant la manche et/ou le pan dorsal au niveau de la ligne de tension peut avoir des propriétés élastiques différentes de celles du matériau constituant le reste du vêtement en dehors de ladite ligne de tension.

### Description des figures.

D'autres avantages et caractéristiques de l'invention apparaîtront mieux à la lecture de la description d'un mode de réalisation préféré qui va suivre, en référence aux dessins annexés, réalisés à titre d'exemples indicatifs et non limitatifs et sur lesquels :
- la figure 1 est une vue de gauche du vêtement avec son utilisateur qui est en action dans une configuration courbée vers le sol, ses bras sont doublés par des sangles situées au niveau de ses flancs,
- la figure 2 est une vue de gauche d'un autre mode de réalisation du vêtement avec son utilisateur qui est en action dans une configuration courbée vers le sol, ses bras sont doublés par une sangle dorsale,
- la figure 3 est une vue de gauche d'un troisième mode de réalisation non revendiqué du vêtement porté à l'envers, avec son utilisateur qui est en action dans une configuration courbée vers le sol, ses bras sont doublés par des sangles situées au niveau des manches,
- la figure 4 est une vue de derrière du vêtement de la figure 1,
- la figure 5 est une vue de derrière du vêtement de la figure 2,
- les figures 6a et 6b représentent un moyen d'accroche permettant de solidariser le vêtement avec la main de l'utilisateur
- la figure 7a est une vue de gauche d'un autre mode de réalisation non revendiqué du vêtement, avec son utilisateur qui est en action dans une configuration courbée vers le sol,
- la figure 7b est une vue de derrière du vêtement de la figure 7a.

### Modes préférés de réalisation de l'invention.

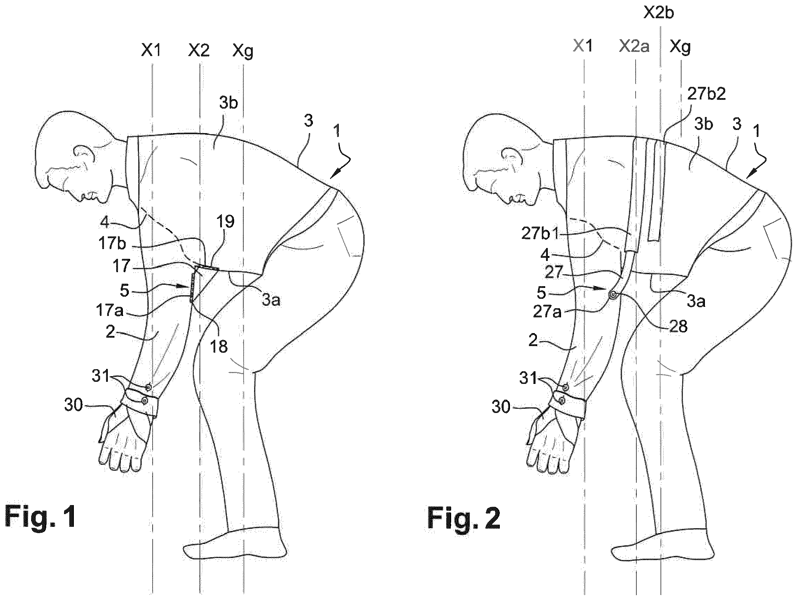

Conformément aux figures 1 à 3, le vêtement, objet de l'invention, comprend une veste **1** comportant un gilet **3** rattaché à deux manches **2** au niveau d'emmanchures **4.** Les manches **2** peuvent être cousues au niveau des emmanchures **4,** ou directement intégrées à la veste **1.** Dans ce dernier cas, les emmanchures **4** sont définies par l'intersection entre les manches **2** et le gilet **3.** Le gilet **3** est composé d'un pan ventral **3a** et d'un pan dorsal **3b.** II comporte un aménagement **5** agencé au niveau de chacune des manches **2** de manière à former une ligne de tension entre un moyen d'accroche **30** situé au niveau des mains de l'utilisateur et le pan dorsal **3b.** Ce vêtement peut comporter sur le pan ventral **3a,** des éléments d'ouverture et fermeture, tels qu'une fermeture éclair, des boutons ou encore une fermeture de type Velcro®. Il habille un utilisateur, comme par exemple un manutentionnaire, et est réalisé en tissu approprié à haute résistance comme par exemple du coton ou un matériau polymère, non élastique ou faiblement élastique, et avec des tailles variées et choisies pour convenir au mieux à l'utilisateur.

Sur la figure 1, l'aménagement **5** présent au niveau de chacune des manches **2** consiste en des sangles **17** qui relient le pan dorsal **3b** du gilet **3** à chacune de ces manches **2.** Ces sangles **17** ont une extrémité distale **17a** rattachée à la manche **2** et une extrémité proximale **17b** rattachée au pan dorsal **3b.** Un moyen d'attache **19,** placé sur le pan dorsal **3b** du gilet **3,** situé à proximité de l'emmanchure **4,** permet de solidariser l'extrémité **17b** de la sangle **17** au gilet **3.** Il peut, par exemple, se trouver sous la forme d'un système de boutons-pressions, d'un fermoir, ou encore d'un système de fixation du genre Velcro ®. L'extrémité **17b** peut aussi être façonnée de manière amovible en forme d'une boucle permettant d'accueillir par exemple, un moyen type attache rapide, ou un mousqueton présent au niveau de la manche. Préférentiellement, cette extrémité **17b** se trouve sous la forme d'un chapelet de boucles permettant d'adapter la longueur des sangles **17** à la morphologie de l'utilisateur.

L'extrémité distale **17a** de la sangle **17** est attachée à la manche **2** au niveau de la partie supérieure de celle-ci, juste en dessous de l'emmanchure **4.** Le moyen d'attache **18** utilisé peut varier et se présenter, par exemple, sous la forme d'une couture, d'un bouton, ou encore d'un système de boucles et d'attache rapide similaire à celui décrit dans le paragraphe précédent. Une fois les deux extrémités **17a, 17b** rattachées respectivement à la manche et au pan dorsal **3b** du gilet **3,** une ligne de tension se créée lorsque l'utilisateur se courbe. Une partie de cette ligne de tension est réalisée grâce à la mise en tension du tissu de la manche **2.** Préférentiellement, le tissu constituant les manches **2** possède des renforcements permettant d'éviter leur déchirure. Cette ligne de tension se trouve dans l'alignement des bras et des sangles **17,** et permet de doubler les bras de l'utilisateur en limitant et/ou bloquant leur allongement, obligeant ainsi l'utilisateur à plier les genoux. Cette sangle **17** permet de reporter tout ou partie de la charge au niveau du pan dorsal **3b.**

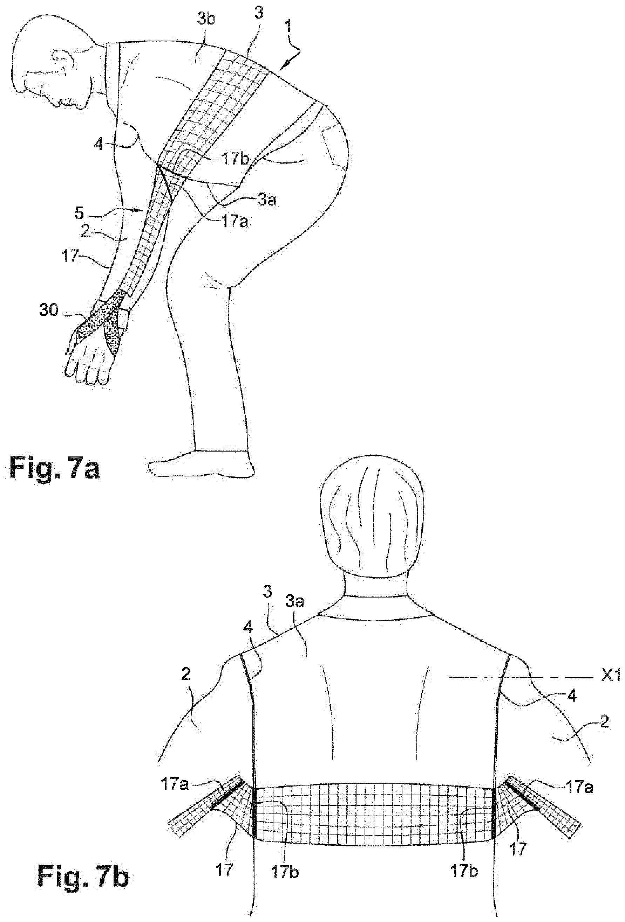

Sur les figures 7a et 7b, la sangle **17** est rapportée sur les manches **2** et/ou le pan dorsal **3b** le long de la ligne de tension décrite précédemment. Un tel mode de réalisation non revendiqué permet de renforcer la solidité du vêtement. La sangle **17** est alors composée d'un matériau aux propriétés élastiques différentes de celles du reste de la veste **1.** La sangle **17** peut se présenter sous différentes formes comme par exemple, une bande de tissu rapportée à la veste **1,** et venant renforcer ou remplacer le tissu de ladite veste **1** au niveau de la ligne de tension. Cette bande de tissu peut être constituée d'une maille ou de fils adaptés pour conférer à la sangle **17** ses propriétés élastiques.

La sangle **17** peut également être directement intégrée dans la veste **1** lors du tricotage du vêtement. Ce mode de réalisation permet d'obtenir un vêtement pour travaux de manutention en une seule pièce et sans couture, étant par conséquent plus solide résistant. La sangle **17,** le long de la ligne de tension, possède, par exemple, des propriétés élastiques différentes de celles du reste du vêtement. Elle peut être formée de mailles et/ou de fils différents de ceux utilisés pour le reste du vêtement. Les fils peuvent être, par exemple, plus épais, et les mailles peuvent, notamment, être plus resserrées. Pour renforcer les effets induits par la ligne de tension, les manches **2** et/ou le pan dorsal **3b,** le long de ladite ligne de tension, peuvent également être fabriqués de manière à avoir des propriétés élastiques différentes, en variant par exemple, le serrage des mailles et/ou le type de fils.

Dans le cas où l'utilisateur ne porterait pas le vêtement, l'axe **X1** de soulèvement de la charge passe au niveau des épaules de l'utilisateur, c'est-à-dire éloigné de son axe de gravité **Xg.** De fait, il existe un bras de levier important entre l'axe **X1** de soulèvement de la charge et l'axe de gravité **Xg.** Ce bras de levier contribue à solliciter le dos et les lombaires, et donc à faire souffrir l'utilisateur.

En se référant à la figure 1, lorsque l'utilisateur porte le vêtement selon l'invention, on constate que le nouvel axe **X2** de soulèvement de la charge passe non plus au niveau des épaules de l'utilisateur mais est déporté vers l'axe de gravité **Xg.** On diminue ainsi le bras de levier, soulageant de ce fait le dos et les lombaires de l'utilisateur. Une ligne de tension est ainsi créée permettant un doublage pour chacun des bras lors de l'usage du vêtement. Lorsque l'utilisateur plie les genoux de manière à soulever une charge, le tissu des manches **2** ainsi que du pan dorsal **3b** se tendent de manière à bloquer l'allongement des bras. Cette caractéristique permet de limiter la tension exercée sur les bras lors du soulèvement de la charge et diminue les contraintes exercées sur l'utilisateur. La charge est alors répartie sur le pan dorsal **3b** et le dos de l'utilisateur, sur une hauteur pouvant être comprise entre 5 cm et 20 cm.

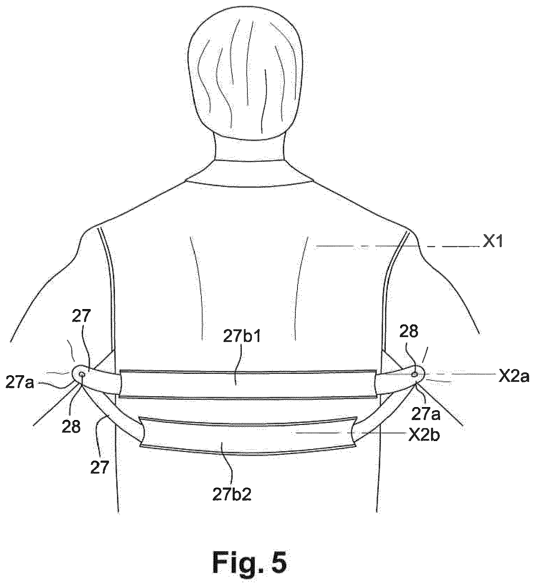

Sur les figures 2 et 5, le vêtement comporte une sangle **27** dont chacune de ses extrémités **27a** est solidaire de chacune des manches **2.** Chaque extrémité **27a** est attachée au niveau de la partie supérieure des manches **2** grâce à des moyens similaires à ceux décrits précédemment. La sangle **27** est glissée dans un fourreau **27b1, 27b2** situé sur le pan dorsal **3b** de manière à l'assembler à la veste **1.** Ce fourreau **27b1, 27b2** est placé sur le pan dorsal **3b** du gilet **3** de manière à ce que la sangle **27** passe sous les omoplates et au niveau des flancs de l'utilisateur. Il est préférentiellement cousu au gilet mais peut être aussi rattaché grâce à des moyens de fixation tels que des boutons ou encore des fermetures éclairs. Il s'étend sur toute la largeur du dos, allant d'un flanc de l'utilisateur à l'autre.

Plusieurs fourreaux sont représentés, l'utilisateur ayant ainsi le choix de glisser la sangle **27** dans l'un ou l'autre des fourreaux **27b1, 27b2** en fonction des tâches qu'il a à accomplir. Bien qu'uniquement deux fourreaux **27b1, 27b2** ne soient représentés sur les figures 2 et 5, il est bien sûr possible d'avoir un vêtement en possédant un plus grand nombre. Suivant un principe similaire à celui décrit précédemment, en usage, la présence de la sangle **27** bloque l'allongement des bras de l'utilisateur et permet ainsi de reporter la charge au niveau du pan dorsal **3b** et donc au niveau du dos de l'utilisateur.

Pour ce mode de réalisation, le principe est similaire à celui décrit pour la figure 1. Une ligne de tension est créée dans les manches **2** ainsi que dans la sangle **27** glissée dans le fourreau **27b1, 27b2.** Suivant le fourreau utilisé, la répartition de la charge se fait sur des positions différentes dans le dos de l'utilisateur. En se référant à la figure 2, suivant le fourreau dans lequel est glissée la sangle, le nouvel axe de soulèvement de la charge **X2a, X2b** peut être plus ou moins déporté vers l'axe de gravité **Xg** de l'utilisateur. Le fourreau est choisi en fonction des mouvements réalisés par l'utilisateur et/ou de l'inclinaison de son buste par rapport au sol.

Sur les figures 1 à 3, 6a et 6b, le vêtement possède des moyens d'accroche **30** permettant de le solidariser avec les mains de l'utilisateur et éviter que les manches ne se relèvent lorsque l'utilisateur soulève une charge. Ce moyen d'accroche **30** peut être attaché au poignet de la veste **1** ou à une sangle **37** (décrite ci-après) en utilisant des moyens d'attache **31.** Ces moyens d'attache **31** peuvent se présenter sous la forme de plusieurs boutons alignés permettant ainsi de régler la position des manches **2** selon la longueur des bras de l'utilisateur. Ils peuvent toutefois se présenter sous des formes différentes telles que des Velcro ®, des fermoirs, ou encore des fermetures éclairs.

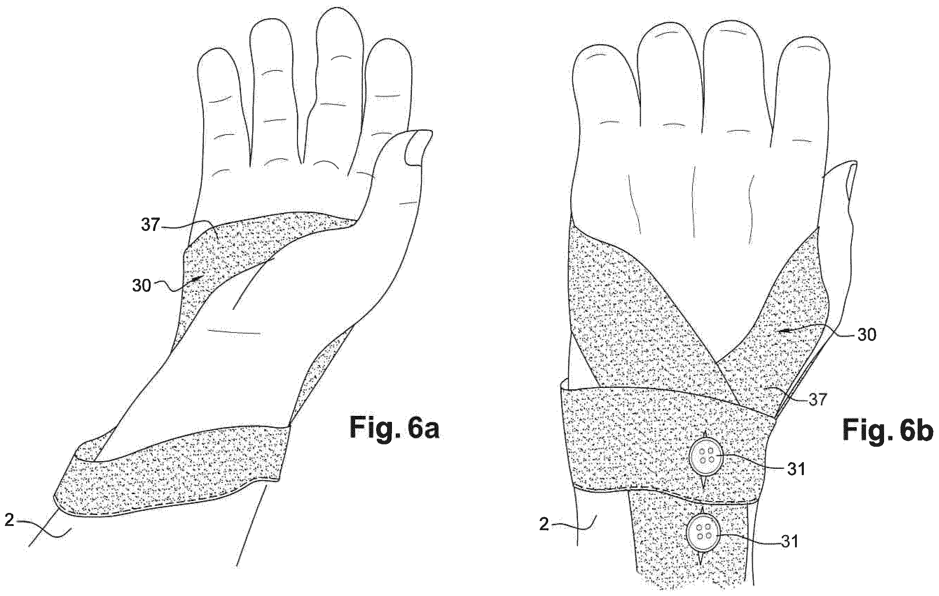

Sur les figures 6a et 6b, le moyen d'accroche **30** se trouve préférentiellement sous la forme d'une boucle venant se coincer au niveau du pouce, permettant ainsi de la faire glisser facilement autour de l'avant-bras de manière à pouvoir remonter les manches **2** lorsque le vêtement n'est pas en usage. Cependant, il peut se présenter sous d'autres formes telles qu'un gant, une mitaine, ou encore une poignée tenue dans le poing fermé de l'utilisateur.

Dans le cas ou le moyen d'accroche **30** se trouve sous la forme d'une boucle, celle-ci peut se présenter sous la forme d'une ou deux sangles élastiques se portant l'une sur l'autre. L'utilisateur peut ainsi choisir l'élasticité nécessaire en fonction de la charge à manipuler.

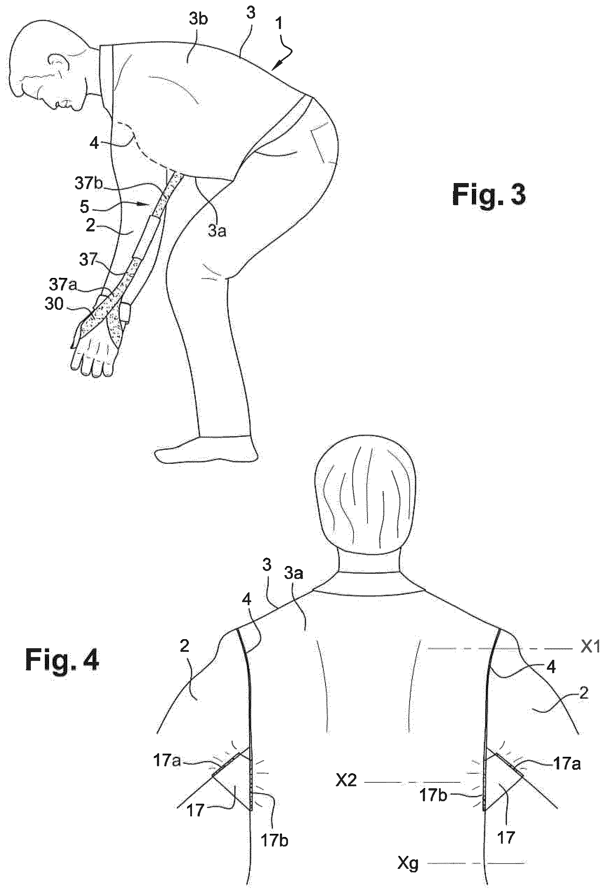

Le mode de réalisation non revendiqué représenté sur la figure 3 peut être indépendant ou supplétif des modes présentés précédemment. Il permet d'augmenter la force de l'utilisateur en permettant de bloquer et/ou limiter l'allongement de ses bras lors de la manutention de charges importantes. L'aménagement **5** situé au niveau des manches **2** consiste en au moins une sangle **37** similaire à celles décrites précédemment. Contrairement aux modes de réalisation décrits ci-dessus, les sangles **37** sont directement reliées aux moyens d'accroche **30** situés sur chacune des mains de l'utilisateur. Cette sangle **37** peut être dans le prolongement soit des sangles **17,** soit de la sangle **27** décrites dans les modes de réalisation précédents. Elle peut toutefois se présenter sous la forme d'une sangle **37** indépendante.

Dans le cas où la sangle **37** est distincte des sangles **17** ou de la sangle **27,** son extrémité proximale **37a** est rattachée au moyen d'accroche **30** situé au niveau des mains de l'utilisateur. Son extrémité distale **37b** est attachée à la partie supérieure de la manche **2,** juste en dessous de l'emmanchure **4.** Les moyens d'attache **31** sont similaires à ceux déjà décrits et peuvent, par exemple, se trouver sous la forme d'un système de boutons-pressions, d'un fermoir, d'un système de fixation du genre Velcro ® ou encore d'une boucle permettant d'accueillir un moyen type attache rapide, ou un mousqueton.

Dans le cas où la sangle **37** se trouve soit dans le prolongement des sangles **17,** soit dans celui de la sangle **27,** son extrémité proximale **37a** est toujours reliée au moyen d'accroche **30** alors que son extrémité distale **37b** est reliée à l'extrémité **17a, 27a** des sangles **17, 27.**

Pour une meilleure efficacité, la sangle **37** est préférentiellement solidarisée à la manche **2.** Celle-ci peut être solidarisée grâce à des moyens pouvant se trouver sous la forme d'un fourreau (figure 3) dans lequel la sangle **37** se glisse. Il peut toutefois se présenter sous d'autres formes, comme par exemple plusieurs boucles dans lesquelles s'insèrent la sangle **37,** ou encore des mousquetons.

Dans ce mode de réalisation, en plus des mécanismes décrits pour les modes des figures 1 et 2, en usage, les sangles **37** vont se tendre et permettre d'améliorer la ligne de tension créée en reportant la tension des manches **2** aux sangles **37.** Ce mode de réalisation permet ainsi la manutention de charges plus importantes. L'effort effectué par l'utilisateur est alors transféré des mains vers les lombaires.

L'agencement des différents éléments et/ou moyens et/ou étapes, dans les modes de réalisation décrits ci-dessus, ne doit pas être compris comme exigeant un tel agencement dans toutes les implémentations. En tout état de cause, on comprendra que diverses modifications peuvent être apportées à ces éléments et/ou moyens et/ou étapes. En particulier :
- les moyens d'accroche **30** peuvent se trouver sous différentes formes,
- les sangles **17, 27, 37** peuvent être élastiques ou non,
- le nombre de fourreaux **27b1, 27b2** peut varier d'un vêtement à un autre,
- les tissus constituant le vêtement peuvent être différents,
- les dispositifs **18, 19, 29** peuvent se présenter sous des formes différentes,
- le vêtement peut être fabriqué dans des tailles différentes,
- les sangles **37** peuvent être rattachées ou non aux sangles **17, 27,**
- les sangles **37** peuvent être utilisées avec ou indépendamment des sangles **17,27,**
- le moyen permettant la solidarisation des sangles **37** avec la manche **2** peut varier.

## Revendications

1. Vêtement pour travaux de manutention consistant en une veste (1) comportant deux manches (2) et un gilet (3), lequel gilet (3) se compose d'un pan ventral (3a) et d'un pan dorsal (3b) reliés auxdites manches (2) par des emmanchures (4), lesdites manches (2) comportant chacune une extrémité pourvue d'un moyen d'accroche (30) permettant leur solidarisation avec chacune des mains d'un utilisateur, ledit vêtement comprend au moins un aménagement (5) agencé au niveau de chaque manche (2) de manière à former une ligne de tension entre le moyen d'accroche (30) et le pan dorsal (3b), de sorte que ladite ligne de tension, en usage, oppose et/ou limite l'allongement du bras de l'utilisateur portant ledit vêtement,
**caractérisé en ce que** l'aménagement (5) se compose d'au moins une sangle (17, 27) comportant une extrémité distale (17a, 27a) solidarisée à la manche (2), juste en dessous de l'emmanchure (4), et une extrémité proximale (17b, 27b1, 27b2) aménagée sur le pan dorsal, de sorte qu'une partie de la ligne de tension soit réalisée grâce à la mise en tension du tissu de la manche (2).

2. Vêtement selon la revendication 1, **caractérisé en ce que** l'extrémité proximale (17b) de la sangle (17) est rattachée au pan dorsal (3b), au niveau des flancs de l'utilisateur, de sorte que la ligne de tension se créée lorsque l'utilisateur se courbe.

3. Vêtement selon la revendication 1, **caractérisé en ce que** la sangle (27) est glissée dans un fourreau (27b1, 27b2) aménagé sur le pan dorsal (3b), de manière à ce que ladite sangle (27) passe sous les omoplates et au niveau des flancs de l'utilisateur.

4. Vêtement selon la revendication 3, **caractérisé en ce que** plusieurs fourreaux (27b1, 27b2) sont aménagés sur le pan dorsal (3b).

5. Vêtement selon l'une des revendications 1 à 4, **caractérisé en ce que** la sangle (17, 27) est élastique.

6. Vêtement selon l'une des revendications 1 à 5, **caractérisé en ce que,** en usage, l'aménagement (5) est configuré de sorte que la ligne de tension agit au niveau du pan dorsal (3b) de manière à obtenir une répartition dorsale de la charge autour et/ou à proximité de l'axe de gravité (Xg) de l'utilisateur.

7. Vêtement selon l'une des revendications 1 à 6, **caractérisé en ce que** le moyen d'accroche (30) présente une boucle adaptée pour se coincer sous le pouce de l'utilisateur.

8. Vêtement selon la revendication 7, **caractérisé en ce que** la boucle (30) se compose d'au moins une sangle élastique.

9. Vêtement selon l'une des revendications 7 ou 8, **caractérisé en ce que** la boucle (30) est dimensionnée de manière à pouvoir passer autour de l'avant bras et/ou du bras de l'utilisateur.

10. Vêtement selon l'une des revendications 1 à 9, **caractérisé en ce que** le matériau constituant la manche (2) et/ou le pan dorsal (3b) au niveau de la ligne de tension a des propriétés élastiques différentes de celles du matériau constituant le reste du vêtement en dehors de ladite ligne de tension.

11. Vêtement selon la revendication 10, **caractérisé en ce que** le matériau constituant la manche (2) et le pan dorsal (3b) au niveau de la ligne de tension est moins élastique que le matériau constituant le reste du vêtement en dehors de ladite ligne de tension.

12. Vêtement selon la revendication 1, **caractérisé en ce que** la sangle (17) est directement intégrée dans la veste (1) lors du tricotage dudit vêtement.

13. Vêtement selon la revendication 12, **caractérisé en ce que** la sangle (17), le long de la ligne de tension, possède des propriétés élastiques différentes de celles du reste dudit vêtement.

14. Vêtement selon l'une des revendications 12 ou 13, **caractérisé en ce que** la sangle (17) est formée de mailles et/ou de fils différents de ceux utilisés pour le reste dudit vêtement.

15. Vêtement selon l'une des revendications 12 à 14, **caractérisé en ce que** le matériau constituant la manche (2) et/ou le pan dorsal (3b) au niveau de la ligne de tension a des propriétés élastiques différentes de celles du matériau constituant le reste du vêtement en dehors de ladite ligne de tension.

## Patentansprüche

1. Kleidungsstück für Handhabungsarbeiten, das aus einer Jacke (1) besteht, die zwei Ärmel (2) und eine Weste (3) aufweist, wobei die Weste (3) aus einem Bauchteil (3a) und einem Rückenteil (3b) besteht, die mit den Ärmeln (2) durch Armlöcher (4) verbunden sind, wobei die Ärmel (2) je ein Ende aufweisen, das mit einer Verankerungseinrichtung (30) versehen ist, welche ihre feste Verbindung mit jeder der Hände eines Benutzers ermöglicht, wobei das Kleidungsstück mindestens eine Ausstattung (5) enthält, die im Bereich jedes Ärmels (2) angeordnet ist, um eine Spannungslinie zwischen der Verankerungseinrichtung (30) und dem Rückenteil (3b) zu bilden, damit die Spannungslinie im Gebrauch der Streckung des Arms des das Kleidungsstück tragenden Benutzers entgegenwirkt und/oder sie begrenzt,
**dadurch gekennzeichnet, dass** die Ausstattung (5) aus mindestens einem Gurt (17, 27) besteht, der ein distales Ende (17a, 27a), das direkt unter dem Armloch (4) fest mit dem Ärmel (2) verbunden ist, und ein proximales Ende (17b, 27b1, 27b2) aufweist, das auf dem Rückenteil angeordnet ist, so dass ein Teil der Spannungslinie mit Hilfe des Spannens des Stoffs des Ärmels (2) hergestellt wird.

2. Kleidungsstück nach Anspruch 1, **dadurch gekennzeichnet, dass** das proximale Ende (17b) des Gurts (17) am Rückenteil (3b) im Bereich der Seiten des Benutzers befestigt ist, so dass die Spannungslinie entsteht, wenn der Benutzer sich krümmt.

3. Kleidungsstück nach Anspruch 1, **dadurch gekennzeichnet, dass** der Gurt (27) in ein Futteral (27b1, 27b2) geschoben wird, das auf dem Rückenteil (3b) angeordnet ist, so dass der Gurt (27) unter den Schulterblättern und im Bereich der Seiten des Benutzers durchgeht.

4. Kleidungsstück nach Anspruch 3, **dadurch gekennzeichnet, dass** mehrere Futterale (27b1, 27b2) auf dem Rückenteil (3b) angeordnet sind.

5. Kleidungsstück nach einem der Ansprüche 1 bis 4, **dadurch gekennzeichnet, dass** der Gurt (17, 27) elastisch ist.

6. Kleidungsstück nach einem der Ansprüche 1 bis 5, **dadurch gekennzeichnet, dass** die Ausstattung (5) im Gebrauch so konfiguriert ist, dass die Spannungslinie im Bereich des Rückenteils (3b) wirkt, um eine Rückenverteilung der Last um die und/oder in der Nähe der Schwerpunktachse (Xg) des Benutzers zu erhalten.

7. Kleidungsstück nach einem der Ansprüche 1 bis 6, **dadurch gekennzeichnet, dass** die Verankerungseinrichtung (30) eine Schlaufe aufweist, die geeignet ist, sich unter dem Daumen des Benutzers einzuklemmen.

8. Kleidungsstück nach Anspruch 7, **dadurch gekennzeichnet, dass** die Schlaufe (30) aus mindestens einem elastischen Gurt besteht.

9. Kleidungsstück nach einem der Ansprüche 7 oder 8, **dadurch gekennzeichnet, dass** die Schlaufe (30) so bemessen ist, dass sie um den Vorderarm und/oder den Arm des Benutzers gehen kann.

10. Kleidungsstück nach einem der Ansprüche 1 bis 9, **dadurch gekennzeichnet, dass** das den Ärmel (2) und/oder das Rückenteil (3b) im Bereich der Spannungslinie bildende Material andere elastische Eigenschaften hat als diejenigen des Materials, das den Rest des Kleidungsstücks außerhalb der Spannungslinie bildet.

11. Kleidungsstück nach Anspruch 10, **dadurch gekennzeichnet, dass** das den Ärmel (2) und das Rückenteil (3b) im Bereich der Spannungslinie bildende Material weniger elastisch ist als das den Rest des Kleidungsstücks außerhalb der Spannungslinie bildende Material.

12. Kleidungsstück nach Anspruch 1, **dadurch gekennzeichnet, dass** der Gurt (17) beim Wirken des Kleidungsstücks direkt in die Jacke (1) integriert wird.

13. Kleidungsstück nach Anspruch 12, **dadurch gekennzeichnet, dass** der Gurt (17) entlang der Spannungslinie andere elastische Eigenschaften besitzt als diejenigen des Rests des Kleidungsstücks.

14. Kleidungsstück nach einem der Ansprüche 12 oder 13, **dadurch gekennzeichnet, dass** der Gurt (17) von Maschen und/oder Fäden gebildet wird, die sich von denjenigen unterscheiden, die für den Rest des Kleidungsstücks verwendet werden.

15. Kleidungsstück nach einem der Ansprüche 12 bis 14, **dadurch gekennzeichnet, dass** das den Ärmel (2) und/oder das Rückenteil (3b) im Bereich der Spannungslinie bildende Material andere elastische Eigenschaften hat als diejenigen des Materials, das den Rest des Kleidungsstücks außerhalb der Spannungslinie bildet.

## Claims

1. Garment for handling work, consisting of a jacket (1) comprising two sleeves (2) and a waistcoat (3), which waistcoat (3) is made up of a front panel (3a) and of a back panel (3b) which are connected to the said sleeves (2) by armholes (4), the said sleeves (2) each having one end provided with an attachment means (30) allowing them to be secured to each of the hands of a user, the said garment comprising at least one arrangement (5) arranged on each sleeve (2) in such a way as to form a tension line between the attachment means (30) and the back panel (3b) so that the said tension line, in use, opposes and/or limits the straightening of the arm of the user wearing the said garment,
**characterized in that** the arrangement (5) is made up of at least one strap (17, 27) comprising a distal end (17a, 27a) secured to the sleeve (2), just below the armhole (4) and a proximal end (17b, 27b1, 27b2) arranged on the back panel, so that part of the tension line is achieved by the tensioning of the fabric of the sleeve (2).

2. Garment according to Claim 1, **characterized in that** the proximal end (17b) of the strap (17) is attached to the back panel (3b), at the level of the user's sides, so that the tension line is created when the user bends.

3. Garment according to Claim 1, **characterized in that** the strap (27) is slipped into a casing (27b1, 27b2) arranged on the back panel (3b), so that the said strap (27) passes underneath the shoulder blades and at the level of the user's sides.

4. Garment according to Claim 3, **characterized in that** several casings (27b1, 27b2) are arranged on the back panel (3b).

5. Garment according to one of Claims 1 to 4, **characterized in that** the strap (17, 27) is elastic.

6. Garment according to one of Claims 1 to 5, **characterized in that**, in use, the arrangement (5) is configured in such a way that the tension line acts on the back panel (3b) so as to distribute the load on the back around and/or near the user's axis of gravity (Xg).

7. Garment according to one of Claims 1 to 6, **characterized in that** the attachment means (30) has a loop designed to wedge under the user's thumb.

8. Garment according to Claim 7, **characterized in that** the loop (30) is made up of at least one elastic strap.

9. Garment according to one of Claims 7 and 8, **characterized in that** the loop (30) is sized so that it can fit around the user's forearm and/or arm.

10. Garment according to one of Claims 1 to 9, **characterized in that** the material of which the sleeve (2) and/or the back panel (3b) is made in the region of the tension line has elastic properties different from those of the material of which the rest of the garment is made away from the said tension line.

11. Garment according to Claim 10, **characterized in that** the material of which the sleeve (2) and the back panel (3b) are made in the region of the tension line is less elastic than the material of which the rest of the garment away from the said tension line is made.

12. Garment according to Claim 1, **characterized in that** the strap (17) is directly built into the jacket (1) during the knitting of the said garment.

13. Garment according to Claim 12, **characterized in that** the strap (17), along the tension line, has elastic properties different from those of the rest of the said garment.

14. Garment according to one of Claims 12 and 13, **characterized in that** the strap (17) is formed of stitches and/or threads different from those used for the rest of the said garment.

15. Garment according to one of Claims 12 to 14, **characterized in that** the material of which the sleeve (2) and/or the back panel (3b) are made in the region of the tension line has elastic properties different from those of the material of which the rest of the garment is made away from the said tension line.
